# EUROPEAN PATENT SPECIFICATION

(11) **EP 2 809 305 B1**
(45) Date of publication and mention of the grant of the patent: **07.10.2015**
(21) Application number: 13714360.8
(22) Date of filing: 28.01.2013
(51) Int. Cl.: A61K 9/24, A61K 9/20, A61K 31/19, A61K 31/70

(54) **BILAYER TABLET FORMULATIONS OF FLURBIPROFEN AND GLUCOSAMIN**
ZWEISCHICHTIGE TABLETTENFORMULIERUNGEN AUS FLURBIPROFEN UND GLUCOSAMIN
FORMULATIONS POUR COMPRIMÉS BICOUCHES DE FLURBIPROFÈNE ET GLUCOSAMINE

(30) Priority: 31.01.2012 TR 201201091; 17.08.2012 TR 201209601
(43) Date of publication of application: 10.12.2014
(73) Proprietor: Sanovel Ilac Sanayi Ve Ticaret Anonim Sirketi, 34460 Istanbul (TR)
(72) Inventor: CIFTER, Umit, 34460 Istanbul (TR); TURKYILMAZ, Ali, 34460 Istanbul (TR); MUTLU, Onur, 34460 Istanbul (TR)
(74) Representative: Sevinç, Erkan
(86) International application number: PCT/TR2013/000045
(87) International publication number: WO 2013/115736

(56) References cited:
- WO-A1-98/52545
- WO-A2-2008/008474
- US-A1- 2009 074 889
- DATABASE WPI Week 200681 Thomson Scientific, London, GB; AN 2006-792937 XP002710728, & JP 2006 290812 A (ROHTO SEIYAKU KK) 26 October 2006 (2006-10-26)

## Description

### Field of Invention

The present invention relates to a bilayer tablet formulation of flurbiprofen and glucosamine. Particularly, the present invention relates to a bilayer tablet formulation having a controlled release flurbiprofen and immediate release glucosamine; furthermore relates to their process and use.

### Background of Invention

Osteoarthritis is the most prevalent form of arthritis, one of the most common diseases affecting humans and a common cause of disability. It is characterized by pain and progressive degeneration of cartilage in synovial joints and vertebrae, leading to significant reduction of mobility and quality of life. Hence, pharmacological treatment of arthritis involves two therapeutic goals:
- Analgesic & anti-inflammatory treatment: Relief from pain and inflammation of the soft tissue surrounding the joint.
- Disease-modifying treatment to treat the underlying pathology

Flurbiprofen is a well known, propionic acid derivative, also known as NSAID (non-steroidal anti-inflammatory drug), with the analgesic and anti-inflammatory activities it possesses. It is used in muscle-skeletal and joint disorders such as ankylosing spondylitis, osteoarthritis and rheumatoid arthritis, in soft-tissue disorders such as sprains and strains and for postoperative pains and mild to moderate pain including dysmenorrhoea and migraine. Its chemical structure is illustrated with Formula I given below.

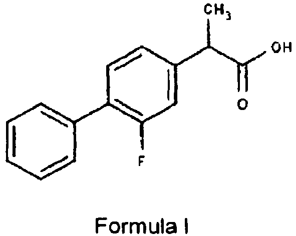

Flurbiprofen is mostly administrated orally in dosages about 100 to 200 mg, may also be increased to 300 mg daily in acute or severe conditions if necessary. One disadvantage of the oral administration of flurbiprofen comprising compositions is that the patient is likely to experience unpleasant side effects, including gastrointestinal (GI) adverse effects including inflammation, spontaneous gastric bleeding, ulceration and perforation of the stomach, which can be life threatening. Thus, using flrubiprofen in high dosages may increase the GI adverse effects.

Another disadvantage of flurbiprofen is that it is practically insoluble in water. This makes it difficult to prepare controlled release formulations.

Glucosamine is an amino sugar and aprominent precursor in the biochemical synthesis of glycosylated proteins and lipids. Glucosamine is part of the structure of the polysaccharides chitosan and chitin and it is naturally present in the shells of shellfish, animal bones and bone marrow. It is also present in some fungi and can be also synthetically derived. Glucosamine is used for the treatment of osteoarthritis. Glucosamine may be administered in dosages about 500 to 2500 mg per day.

The solubility and dissolution rate of glucosamine may influence the bioavailability of flurbiprofen. For this reason, it is quite important to increase the solubility and dissolution rate of these active agents independently.

Also, it is known that preparing the formulations of two different actives having different release profiles is difficult to process and may require complex design of tablet formulations. Accordingly, it is required to show the release profiles at the same time to eliminate the undisered side effects, especilally the GI adverse effects of flurbiprofen. Thus, it is important to provide the efficiency of the formulation by providing a tablet formulation which helps to dissolve the both actives in same manner and same time.

Another problem is related to combine these two active ingredients in one dosage form such as tablet or capsule, it would require a dosage form having approximately or more than 1000 mg active ingredients in total without any further tablet or capsule excipients. This is an amount that would create a very large tablet or capsule size that would not be swallowable, or it would require formulation that would require ingesting multiple tablets to achieve the desired effect. Moreover, it is known that to have a desired sustained release formulations a large amount of polymers are needed to provide the release rate in sustained form such as at least 20 % by weight of the total tablet weight. This makes the tablet size even greater. Thus, a good tablet design and process methods for obtaining them therefore still needed. Another problem in relation to these active agents is stability, which emerges under the influence of ambient and physical conditions, as is the case with many other active agents. They are highly-susceptible to air and humidity. When they are exposed to air and humidity, they degrade structurally and develop chemical behavioral changes. The stability of the products developed may not at a desired level and the shelf life thereof may be shortened. In addition, these active agents are reactive against the excipients employed in developing the formulations containing the same. This, in turn, may cause impurities to occur in the formulations and may lead to the inclusion of undesired components into the formulations.

There are various patent applications in prior art in relation to glucosamine formulations but none of them are specifically used in combination with flurbiprofen in oral administration as tablet dosage form.

For example, US 2008/0227747 A1 discloses a therapeutic composition and methods for the treatment and prevention of a degenerative joint disorder and/or cardiovascular disease comprising polycosanols, glucosamine and chondroitin. Composition further may comprise NSAIDs, but neither an example nor flurbiprofen as one of the NSAIDs is disclosed in the patent application in combination with glucosamine, and the US application is silent about the problems related to its formulation and manufacturing process.

There remains a need for to provide bioavailable, stable and easily processed pharmaceutical bilayer tablet formulations of flurbiprofen having a sustained release and glucosamine having an immediate relase profile which overcomes the problems described above.

Further advantages and embodiments of the present invention will become apparent from the following description.

### Summary of the Invention

The object of the present invention is, therefore to provide bioavailable, stable and easily processed pharmaceutical bilayer tablet formulations of flurbiprofen having a sustained rlease in combination with glucosamin having an immediate relase profile that brings advantages over them.

Accordingly, the object of the present invention is to obtain a stable formulation with having a desired solubility and dissolution rate, and therefore a desired level of bioavailability. Another object of the invention is to provide reduced dosage regimens for the outpatients by providing a sustained release formulation as described in claim 1. For example, the reduction of a dose regimen from four times a day to three times a day allows the patient to take the prescribed drug during waking hours. Reduction of a dose regimen to twice a day allows the patient to take the prescribed drug in the morning and in the evening, which provides greater convenience; e.g., the patient is not required to carry an additional one when away from home. Of course, the most convenient dosage form is a once daily dose regimen. This also reduces the risk of the omitted tablets. Therefore, better assurance of compliance and convenient dosage regimens are provided for outpatients by the present invention. According to present invention the dosage regimen is once-a-day oral administration.

A further object of the invention is to eliminate the GI adverse effects of flurbiprofen when it is administered orally. It is known that to formulate a sustained release formulation requires the use of high terapeutic effective amounts, thus this may increase the GI adverse effects. The present invention provides the solution to this problem by using rate controlling polymers with flurbiprofen, therefore the release of the flurbiprofen is controlled and desired relase is obtained in gastro-intestinal track.

The pharmaceutical formulation of this invention advantageously provides a bilayer tablet dosage form in sandwich form which is bioequivalent to a capsule dosage form of the same or substantially similar strength. Moreover, the required amount of rate controlling polymer to obtain a sustained release formulation is used less than 20 % by weight of the total tablet, which is known at least this amount (20 %) is sufficient in prior art to get the sustained release. Therefore it has been achieved only using small amount of excipients which help the formulation in required release and easily processed into a layered tablet form, in desired weight which can easily be swallowed by the patients. This also prevents the dose dumping of the active ingredient which can be a serious problem caused by the wrong design of the modified release formulations which will be discussed further in detailed description.

A pharmaceutical formulation is developed to carry out all objects, referred to above and further advantages and embodiments of the present invention will become apparent from the following description

### Detailed Description of Invention

As used herein, "sustained release dosage forms" are defined as those whose drug release characteristics of time course and/or location are chosen to accomplish therapeutic or convenience objectives not offered by conventional forms. A sustained release dosage form potentially provides greater effectiveness in the treatment of chronic conditions; greater convenience; reduces side effects and provides higher levels of patient compliance or therapeutic performance due to a simplified dosage schedule, compared with those of immediate-release drugs. Sustained release pharmaceutical products are formulated to release the drug's active ingredient gradually and predictably over a 12-hour to 24-hour period.

As used herein, "rate controlling agent" means an excipient in the first layer of the final dosage form whose primary function is to modify the duration of release of the active drug substance from the dosage form.

Flurbiprofen useful in accordance with this invention comprises the pharmaceutically acceptable salts and esters of flurbiprofen, and further includes the conventionally used racemic mixture which comprises the S- and R- enantiomers of flurbiprofen. In a preferred embodiment of the present invention, flurbiprofen is in an amount of 5.0 to 25.0 % by weight of the total tablet, preferably it is 10.0 to 20.0 % by weight of the total tablet.

The preferred salts of glucosamine in accordance with this invention comprise N-acetylglucosamine, glucosamine hydrochloride and glucosamine sulfate and mixtures thereof. In a preferred embodiment of the present invention, glucosamine or salts thereof is in an amount of 25.0 to 80.0 % % by weight of the total tablet, preferably it is 35.0 to 70.0 % by weight of the total tablet, more preferably it is 45.0 % to 65.0 % by weight of the total tablet.

According to the present invention, a novel bilayer tablet formulation comprising, a first layer comprising flurbiprofen wherein said first layer allows sustained release of flurbiprofen and a second layer comprising glucosamine or salts thereof wherein said second layer allows immediate release of glucosamine is obtained wherein said bilayer tablet is in sandwich form.

According to preferred embodiment, the present invention provides a bilayer tablet in sandwich form which means one layer is located on the other layer. This helps both molecules to disintegrate independently from each other from both surfaces of the layered tablet when taken into the body. Because of their location on the outer layers of the tablet flurbiprofen and glucosamin can easily disintegrate and disolve without effected any other coating or layers. Thus, the dissolution profile of the actives (flurbiprofen and glucosamine) are surprisingly increased and the desired bioavailabilty is obtained. In prior art most of the layered tablets are designed differently such as the active is located in the core and other layers are located on the core as outer shell which surrounds it, they also called compression coated tablets. This may cause dissolution problems or retardant effect more than the desired one. Also the layered tablet is more preferably over the other tablet categories such as compression coated tablets as the surface contact is less and the production is simple and more rapid.

Another disadvantage of the compression coated tablets is that, this tablet readily lend itself into a repeat action tablet as the outer layer provides the initial dose while the inner core release the drug later on. But, when the core quickly releases the drug, entirely different blood level is achieved with the risk of over dose toxicity. This may cause dose dumping of the active ingredients. Dose dumping is one of the most important disadvantages of sustained release dosage forms. Because of several different reasons it is difficult to develop sustained release formulations and they can be prone to "dose dumping" in which the release of the active ingredient is delayed but once the release begins the medicament is released very fast. The most important criteria of dose dumping under in-vitro conditions, is the amount of the active substance released in early time point. In prior art, to prevent dose dumping rate controlling polymers are used in general but the sufficient amount is greater than about 20% (w/w) of the dosage form and in a weight ratio greater than 1:1 relative to the drug. On the other hand using high amount of polymers may cause other problems.

Therefore, the present invention provides the solution to this problem by formulating a bilayer tablet in sandwich form which helps to separate the active ingredients immediately with the uptake into the body and eliminate the use of high excipients. Accordingly the rate controlling polymers used in this present invention is only max. 5.0% by weight of the total tablet weight which is lower than the ones used in prior art.

Therefore, the release profiles of the actives are as follows; the maximum 40% of total amount of flurbiprofen is released in 2 hours and 50 - 80% in 6 hours and at least 75% in 8 hours in 900 ml of phosphate buffer at pH 7.2 at 37°C using USP paddle method rotating at 50 RPM. Glucosamine is released immediately and minimum 75% of the total amount is dissolved in 60 min.

According to a preferred embodiment of the present invention, said first layer comprising rate controlling polymers selected from the group comprising polymethacrylates, particularly ammonio methacrylate copolymers, methacrylic acid-ethyl acrylate copolymer, methacrylic acid-methyl methacrylate copolymer, cationic methacrylate, polyethylene glycol, cellulose acetate phthalate, acetylated monoglyceride, dibutyl tartrate, diethyl phthalate, dimethyl phthalate, glycerin, propylene glycol and tripropionin or their mixtures.

According to a preferred embodiment of the present invention, said rate controlling polymers are in an amount of 0.5 to 5.0 % by weight of the total tablet and preferably said rate controlling polymer is ammonio methacrylate copolymers.

According to preferred embodiment, the present invention provides enhanced solubility of glucosamin by using at least a disintegrant in second layer of the bilayer tablet. Suitable disintegrants, may comprise but not limited to croscarmellose sodium, xylitol, polyplasdone (1-ethenylpyrrolidin-2-one), crospovidone, low-substituted hydroxypropyl cellulose (L-HPC) and sodium starch glycolate or mixtures thereof. According to this preferred embodiment, said disintegrants are in an amount of 0.5 to 5.0 % by weight of the total tablet and preferably said disintegrant is crosscarmellose sodium.

The pharmaceutical compositions according to the present invention may also comprise one or more pharmaceutically acceptable excipients. In a preferred embodiment, pharmaceutically acceptable excipients are selected from the group comprising binders, fillers, glidants, lubricants or mixtures thereof.

Suitable binders, may comprise but not limited to polyvinylpyrrolidone, hydroxsypropyl methyl cellulose, hydroxsypropyl cellulose, carboxy methyl cellulose, methyl cellulose, hydroxy ethyl cellulose, carboxy methyl cellulose sodium, carboxymethyl cellulose calsium, ethyl cellulose, polyethylene oxide, gelatin, starch, xanthan gum, guar gum, alginate, carrageenan, pectin, carbomer, cellulose acetat phytalate, hydroxy propyl starch, polaxomer, poly ethylene glychol or mixtures thereof. The preffered binders of the present invention are polyvinylpyrrolidone, poly ethylene glychol or mixtures thereof. According to this preferred embodiment, said binders are in an amount of 0.5 to 5.0 % by weight of the total tablet.

Suitable fillers, may comprise but not limited to microcrystalline cellulose, lactose monohydrate, mannitol, starch, sugars, sorbitol, sucrose and mixtures thereof. Preferably the fillers are microcrystalline cellulose, lactose monohydrate or mixtures thereof. According to this preferred embodiment, said fillers are in an amount of 1.0 to 15.0 % by weight of the total tablet.

Furthermore, in this present invention, a stable formulation is surprisingly obtained which has a high solubility and dissolution rate. Said formulation comprises flurbiprofen in first layer and glucosamine in second layer and a glidant at least in one layer which improves the stabilization during the process. Suitable glidants, may comprise but not limited to colloidal silicon dioxide, talc or mixtures thereof. Preferably the glidant is colloidal silicon dioxide.

Suitable lubricants, may include but not limited to magnesium stearate, sodium stearyl fumarate, polyethylene glycol, stearic acid, metal stearates, boric acid, sodium chloride benzoate and acetate, sodium or magnesium lauryl sulfate or mixtures thereof. Preferably the lubricant selected for the invention is magnesium stearate.

According to a further embodiment of the invention, first layer may comprise plasticizer which is selected from the group comprising citrate esters such as acetyl tributyl citrate, acetyl triethyl citrate, or triethyl citrate; phthalate esters such as diethyl phthalate or dibutyl phthalate; fatty acid esters such as butly stearate, glycerol monostearate or stearyl alcohol; dibutyl sebacate, triacetine, castor oil, glycerin or mixtures thereof. Preferably, said plasticizer is triethyl citrate. Plasticizers protect the pellets from breaking and increase the elasticity, furthermore they help to obtain homogenized pellets by decreasing the electrification this helps to prevent the sticking and agglomeration.

In a preferred embodiment according to the present invention, said pharmaceutical formulation comprising,
a. flurbiprofen at 5.0 - 25.0 % by total tablet weight,
   i. ammonio methacrylate copolymer at 0.5 - 5.0 % by total tablet weight,
   ii. polyethylene glycol at 0.01 - 2.0 % by total tablet weight,
   iii. microcrystalline cellulose at 1.0 - 15.0 % by total tablet weight,
   iv. lactose monohydrate at 1.0 - 15.0 % by total tablet weight,
   v. magnesium stearate at 0.01 - 2.0 % by total tablet weight,
b. glucosamine or salts thereof at 25.0 - 80.0 % by total tablet weight,
   i. croscarmellose sodium at 0.5 - 5.0 % by total tablet weight,
   ii. polyvinylpyrrolidone at 0.5 - 5.0 % by total tablet weight,
   iii. microcrystalline cellulose at 1.0 - 15.0 % by total tablet weight,
   iv. lactose monohydrate at 1.0 - 15.0 % by total tablet weight,
   v. colloidal silicon dioxide at 0.01 - 2.0 % by total tablet weight,
   vi. magnesium stearate at 0.01 - 2.0 % by total tablet weight,

In another preferred embodiment according to the present invention, said pharmaceutical formulation comprising,
a. flurbiprofen at 5.0 - 25.0 % by total tablet weight,
   i. ammonio methacrylate copolymer at 0.5 - 5.0 % by total tablet weight,
   ii. neutral pellet at 1.0 - 10.0 % by total tablet weight,
   iii. triethyl citrate at 0.01 - 2.0 % by total tablet weight,
   iv. colloidal silicon dioxide 0.01 - 2.0 % by total tablet weight,
   v. polyethylene glycol and microcrystalline cellulose at 1.0 - 15.0 % by total tablet weight,
   vi. magnesium stearate at 0.01 - 2.0 % by total tablet weight,
b. glucosamine or salts thereof at 25.0 - 80.0 % by total tablet weight,
   i. croscarmellose sodium at 0.5 - 5.0 % by total tablet weight,
   ii. polyvinylpyrrolidone at 0.5 - 5.0 % by total tablet weight,
   iii. microcrystalline cellulose at 1.0 - 15.0 % by total tablet weight,
   iv. lactose monohydrate at 1.0 - 15.0 % by total tablet weight.
   v. colloidal silicon dioxide at 0.01 - 2.0 % by total tablet weight.
   vi. magnesium stearate at 0.01 - 2.0 % by total tablet weight,

Another preferred embodiment according to the present invention provides a method for preparing said pharmaceutical formulation, this method comprising the steps of,
a. preparing the first layer
   i. dissolving polyethylene glycol in water and mixing it with half of the ammonio methacrylate copolymer,
   ii. Flurbiprofen and microcrystalline cellulose is added to high-shear mixture, then solution (i) is added to this mixture and granulated while the mixer is open,
   iii. then granules are dried in fluid bed dryer and sieved,
   iv. the granules obtained in step (iii), are coated with the rest of the ammonio methacrylate copolymer in fluid bed dryer,
   v. lactose monohydrate and magnesium stearate are added to this granules as an external phase.
b. preparing the second layer
   i. glucosamine, microcrystalline cellulose, lactose monhydrate and half of the croscarmellose sodium are mixed together,
   ii. then they are granulated with the water-solution of polyvinylpyrrolidone,
   iii. obtained granules are then sieved and dried in oven at 50 °C,
   iv. dried granules are also sieved and colloidal silicon dioxide, magnesium stearate and rest of the croscarmellose sodium areadded as an external phase.
c. performing a compression step to form the bilayer tablets.

Another preferred embodiment according to the present invention provides a method for preparing said pharmaceutical formulation, this method comprising the steps of,
a. preparing the first layer
   i. water is added to a tank which comprise a mechanic stirrer and a homogenizer (mixture I),
   ii. adding triethly citrate to this tank and mixing it with the mechanic stirrer (mixture II),
   iii. flurbiprofen is added to mixture II while the homogenizer is open to obtain the mixture III,
   iv. ammonio methacrylate copolymer is added to mixture III while the homogenizer is closed and it is mixed until a homogenous mixture is obtained and any bubles remain (mixture IV),
   v. neutral pellets having a mean particle size diameter between 500 to 750 µ are added to fluid bed dryer and the mixture IV is coated on the pellets with spraying,
   vi. after the coating step in v, colloidal silicon dioxide is added on the coated pellets and coated pellets are sieved,
   vii. polyethylene glycol, microcrystalline cellulose and magnesium stearate are added to this granules as an external phase.
b. preparing the second layer
   i. glucosamine, microcrystalline cellulose, lactose monhydrate and half of the croscarmellose sodium are mixed together,
   ii. then they are granulated with the water-solution of polyvinylpyrrolidone,
   iii. obtained granules are then sieved and dried in oven at 50 °C,
   iv. dried granules are also sieved and colloidal silicon dioxide, magnesium stearate and rest of the croscarmellose sodium areadded as an external phase.
c. performing a compression step to form the bilayer tablets.

According to another preferred embodiment of the present invention, the formulation is orally administered as once-a-day dosage regimen.

According to another preferred embodiment of the present invention, the formulation is used for the treatment of osteoarthritis, pain and inflammatory symptoms associated with joint and cartilage disorders.

According to a further embodiment of the present invention, glucosamine may further be combined with chondroitin or methylsulfonylmethane.

This invention is further defined by reference to the following examples. Although the example is not intended to limit the scope of the present invention, it should be considered in the light of the description detailed above.

### Example 1: Bilayer tablet

| **Ingredients** | **amount (mg)** |
|---|---|
| **Controlled release layer** | |
| Flurbiprofen | 200.0 |
| ammonio methacrylate copolymer | 30.7 |
| polyethylene glycol | 1.4 |
| microcrystalline cellulose | 94.6 |
| lactose monohydrate | 50.0 |
| magnesium stearate | 3.3 |

| **Immediate release layer** | |
|---|---|
| glucosamine or salts thereof | 750.0 |
| croscarmellose sodium | 40.0 |
| polyvinylpyrrolidone | 40.0 |
| microcrystalline cellulose | 64.8 |
| lactose monohydrate | 100.0 |
| colloidal silicon dioxide | 1.0 |
| magnesium stearate | 1.2 |

The process of the formulation is carried out as follows:
preparing the first layer: polyethylene glycol is dissolved in water and half of the ammonio methacrylate copolymer is added and mixed together (solution i). Flurbiprofen and microcrystalline cellulose is added to high-shear mixture, then solution (i) is added to this mixture and the mixture is granulated while the mixer is open. Then, granules are dried in fluid bed dryer and sieved. The sieved granules obtained are coated with the rest of the ammonio methacrylate copolymer in fluid bed dryer, finally lactose monohydrate and magnesium stearate are added to this granules as an external phase.
preparing the second layer: glucosamine, microcrystalline cellulose, lactose monhydrate and half of the croscarmellose sodium are mixed together, then they are granulated with the water-solution of polyvinylpyrrolidone. Obtained granules are then sieved and dried in oven at 50 °C. The dried granules are also sieved and colloidal silicon dioxide, magnesium stearate and rest of the croscarmellose sodium are added as an external phase.

Finally, compression step is performed to form the bilayer tablets.

### Example 2: Bilayer tablet

| **Ingredients** | **amount (mg)** |
|---|---|
| **Controlled release layer** | |
| Flurbiprofen | 200.0 |
| Neutral pellet | 68.0 |
| ammonio methacrylate copolymer | 45.88 |
| triethyl citrate | 11.54 |
| Silicon dioxide | 1.58 |
| polyethylene glycol and microcrystalline cellulose | 50.0 |
| magnesium stearate | 3.3 |

| **Immediate release layer** | |
|---|---|
| Glucosamine or salts thereof | 750.0 |
| croscarmellose sodium | 40.0 |
| polyvinylpyrrolidone | 40.0 |
| microcrystalline cellulose | 64.8 |
| lactose monohydrate | 100.0 |
| colloidal silicon dioxide | 1.0 |
| magnesium stearate | 1.2 |

The process of the formulation is carried out as follows:
preparing the first layer: Water is added to a tank which comprise a mechanic stirrer and a homogenizer (mixture I), and triethly citrate is added to this tank and it is mixed with the mechanic stirrer (mixture II). Flurbiprofen is added to mixture II while the homogenizer is open to obtain the mixture III. Ammonio methacrylate copolymer is added to mixture III while the homogenizer is closed and it is mixed until a homogenous mixture is obtained and no bubles remain (mixture IV). Then, neutral pellets having a mean particle size diameter between 500 to 750 µ are added to fluid bed dryer and the mixture IV is coated on the pellets with spraying. After the coating step, colloidal silicon dioxide is added on the coated pellets and coated pellets are sieved. Sieving is important in this step to prevent the coated pellets from sticking to eachother. Finally, polyethylene glycol, microcrystalline cellulose and magnesium stearate are added to this granules as an external phase.
preparing the second layer: Glucosamine, microcrystalline cellulose, lactose monhydrate and half of the croscarmellose sodium are mixed together, then they are granulated with the water-solution of polyvinylpyrrolidone. Obtained granules are then sieved and dried in oven at 50 °C. Dried granules are also sieved and colloidal silicon dioxide, magnesium stearate and rest of the croscarmellose sodium are added as an external phase.

Finally, compression step is performed to form the bilayer tablets.

## Claims

1. A bilayer tablet formulation, comprising
a. a first layer comprising flurbiprofen wherein said first layer allows sustained release of flurbiprofen,
b. a second layer comprising glucosamine or salts thereof wherein said second layer allows immediate release of glucosamine
wherein said bilayer tablet is in sandwich form.

2. The bilayer tablet formulation according to claim 1, wherein said first layer further comprising rate controlling polymers selected from the group comprising polymethacrylates, particularly ammonio methacrylate copolymers, methacrylic acid-ethyl acrylate copolymer, methacrylic acid-methyl methacrylate copolymer, cationic methacrylate, polyethylene glycol, cellulose acetate phthalate, acetylated monoglyceride, dibutyl tartrate, diethyl phthalate, dimethyl phthalate, glycerin, propylene glycol and tripropionin or their mixtures.

3. The bilayer tablet formulation according to claim 2, wherein said rate controlling polymers are in an amount of 0.5 to 5.0 % by weight of the total tablet.

4. The bilayer tablet formulation according to claims 2 and 3, wherein said rate controlling polymer is ammonio methacrylate copolymers.

5. The bilayer tablet formulation according to claim 1, wherein said second layer further comprising disintegrants selected from the group comprising crosscarmellose sodium, crospovidone, low-substituted hydroxypropyl cellulose (L-HPC), sodium starch glycolate, xylitol, polyplasdone (1-ethenylpyrrolidin-2-one) or mixtures thereof.

6. The bilayer tablet formulation according to claim 5, wherein said disintegrants are in an amount of 0.5 to 5.0 % by weight of the total tablet.

7. The bilayer tablet formulation according to claims 5 and 6, wherein said disintegrant is crosscarmellose sodium.

8. The bilayer tablet formulation according to claim 1, wherein flurbiprofen is in an amount of 5.0 to 25.0 % by weight of the total tablet and glucosamine or salts thereof is in an amount of 25.0 to 80.0 % % by weight of the total tablet.

9. The bilayer tablet formulation according to any preceding claims, wherein said layers further comprising pharmaceutically acceptable excipients selected from the group comprising binders, fillers, glidants, lubricants or mixtures thereof.

10. The pharmaceutical formulation according to claim 9, wherein said binder is selected from the group comprising polyvinylpyrrolidone (povidon), hydroxypropyl methyl cellulose, hydroxypropyl cellulose, carboxy methyl cellulose, methyl cellulose, hydroxy ethyl cellulose, carboxy methyl cellulose sodium, carboxymethyl cellulose calcium, ethyl cellulose, polyethylene oxide, gelatin, starch, xanthan gum, guar gum, alginate, carrageenan, pectin, carbomer, cellulose acetat phytalate, hydroxy propyl starch, polaxomer, poly ethylene glychol or mixtures thereof.

11. The bilayer tablet formulation according to claim 10, wherein said binder is polyvinylpyrrolidone (povidon).

12. The pharmaceutical formulation according any preceding claims, wherein first layer may comprise plasticizer which is selected from the group comprising citrate esters such as acetyl tributyl citrate, acetyl triethyl citrate, or triethyl citrate; phthalate esters such as diethyl phthalate or dibutyl phthalate; fatty acid esters such as butly stearate, glycerol monostearate or stearyl alcohol; dibutyl sebacate, triacetine, castor oil, glycerin or mixtures thereof.

13. The bilayer tablet formulation according to claim 12, wherein said plasticizer is triethyl citrate.

14. The pharmaceutical formulation according to claims 1 to 11, comprising,
a. flurbiprofen at 5.0 - 25.0 % by total tablet weight,
i. ammonio methacrylate copolymer at 0.5 - 5.0 % by total tablet weight,
ii. polyethylene glycol at 0.01 - 2.0 % by total tablet weight,
iii. microcrystalline cellulose at 1.0 - 15.0 % by total tablet weight,
iv. lactose monohydrate at 1.0 - 15.0 % by total tablet weight,
v. magnesium stearate at 0.01 - 2.0 % by total tablet weight,
b. glucosamine or salts thereof at 25.0 - 80.0 % by total tablet weight,
i. croscarmellose sodium at 0.5 - 5.0 % by total tablet weight,
ii. polyvinylpyrrolidone at 0.5 - 5.0 % by total tablet weight,
iii. microcrystalline cellulose at 1.0 - 15.0 % by total tablet weight,
iv. lactose monohydrate at 1.0 - 15.0 % by total tablet weight,
v. colloidal silicon dioxide at 0.01 - 2.0 % by total tablet weight,
vi. magnesium stearate at 0.01 - 2.0 % by total tablet weight,

15. The pharmaceutical formulation according to claims 1 to 13, comprising,
a. flurbiprofen at 5.0 - 25.0 % by total tablet weight,
i. ammonio methacrylate copolymer at 0.5 - 5.0 % by total tablet weight,
ii. neutral pellet at 1.0 - 10.0 % by total tablet weight,
iii. triethyl citrate at 0.01 - 2.0 % by total tablet weight,
iv. colloidal silicon dioxide 0.01 - 2.0 % by total tablet weight,
v. polyethylene glycol and microcrystalline cellulose at 1.0 - 15.0 % by total tablet weight,
vi. magnesium stearate at 0.01 - 2.0 % by total tablet weight,
b. glucosamine or salts thereof at 25.0 - 80.0 % by total tablet weight,
i. croscarmellose sodium at 0.5 - 5.0 % by total tablet weight,
ii. polyvinylpyrrolidone at 0.5 - 5.0 % by total tablet weight,
iii. microcrystalline cellulose at 1.0 - 15.0 % by total tablet weight,
iv. lactose monohydrate at 1.0 - 15.0 % by total tablet weight,
v. colloidal silicon dioxide at 0.01 - 2.0 % by total tablet weight,
vi. magnesium stearate at 0.01 - 2.0 % by total tablet weight,

## Patentansprüche

1. Eine Zweischichtentablettenformulierung, umfassend
a. eine erste Schicht umfassend Flurbiprofen, worin die erste Schicht gesteuerte Freisetzung des Flurbiprofens erlaubt,
b. eine zweite Schicht umfassend Glucosamin oder Salze davon, worin die zweite Schicht die sofortige Freisetzung des Glucosamins erlaubt,
wobei die Zweischichtentablette in Sandwichform ist.

2. Die Zweischichtentablettenformulierung gemäß Anspruch 1, worin die erste Schicht weiterhin umfasst ratenkontrollierende Polymere ausgewählt aus der Gruppe, umfassend Polymethacrylat, insbesondere Amonium-Methacrylat-Copolymere, Methacrylsäure-Ethylacrylat-Copolymer, Methacrylsäure-Methylmethacrylat-Copolymer, kationisches Methacrylat, Polyethylenglycol, Cellulose-Acetat-Phthalat, acetyliertes Monoglycerid, Dibutyltartrat, Diethylphthalat, Dimethylphthalat, Glycerin, Propylenglycol und Tripropionin oder deren Mischung.

3. Die Zweischichtentablettenformulierung gemäß Anspruch 2, worin die ratenkontrollierende Polymere in einer Menge von 0,5 bis 5,0 Gew. % der Gesamttablette sind.

4. Die Zweischichtentablettenformulierung gemäß den Ansprüchen 2 und 3, worin das ratenkontrollierende Polymer Amonium-Methacrylat-Copolymere ist.

5. Die Zweischichtentablettenformulierung gemäß Anspruch 1, worin die zweite Schicht weiterhin umfasst Desintegrationsmittel ausgewählt aus der Gruppe, umfassend Crosscarmellose-Natrium, Crospovidon, niedrig substituierte Hydroxypropyl-Cellulose (L-HPC), Natrium-Stärkeglycolat, Xylitol, Polyplasdone (1-Ethenylpyrrolidin-2-on) oder Mischungen davon.

6. Die Zweischichtentablettenformulierung gemäß Anspruch 5, worin die Desintegrationsmittel in einer Menge von 0,5 bis 5,0 Gew. % der Gesamttablette sind.

7. Die Zweischichtentablettenformulierung gemäß den Ansprüchen 5 und 6, wobei das Desintegrationsmittel Crosscarmellose-Natrium ist.

8. Die Zweischichtentablettenformulierung gemäß Anspruch 1, worin Flurbiprofen in einer Menge von 5,0 bis 25,0 Gew. % der Gesamttablette ist und Glucosamin oder Salze davon in einer Menge von 25,0 bis 80,0 Gew. % der Gesamttablette ist.

9. Die Zweischichtentablettenformulierung gemäß irgendeinem der vorhergehenden Ansprüche, worin die Schichten weiterhin umfassen pharmazeutisch annehmbare Hilfsstoffe ausgewählt aus der Gruppe, umfassend Bindemittel, Füllstoffe, Gleitmittel, Schmiermittel oder Mischungen davon.

10. Die pharmazeutische Formulierung gemäß Anspruch 9, worin das Bindemittel ausgewählt ist aus der Gruppe, umfassend Polyvinylpyrrolidone (Povidon), HydroxypropylmethylCellulose, Hydroxypropyl-Cellulose, Carboxymethyl-Cellulose, Methyl-Cellulose, HydroxyethylCellulose, Carboxymethyl-Cellulose-Natrium, Carboxymethyl-Cellulose-Kalzium, EthylCellulose, Polyethylenoxid, Gelatine, Stärke, Xanthan-Gummi, Guar-Gummi, Alginat, Carrageenan, Pectin, Carbomer, Cellulose-Acetat-Phthalat, Hydroxypropylstärke, Polaxomer, Polyethylenglycol oder Mischungen davon.

11. Die Zweischichtentablettenformulierung gemäß Anspruch 10, worin das Bindemittel Polyvinylpyrrolidon (Povidon) ist.

12. Die pharmazeutische Formulierung gemäß einem der vorhergehenden Ansprüche, worin die erste Schicht einen Weichmacher umfassen kann, der aus der Gruppe ausgewählt ist, umfassend Citrat-Ester, wie beispielsweise Acytyl-Tributylcitrat, Acetyl-Triethylcitrat, oder Triethylcitrat; Phthalat-Ester wie beispielsweise Diethyphthalat oder Dibutylphthalat; Fettsäure-Ester wie beispielsweise Butylstearat, Glycerol-Monostearat oder Stearylalkohol; Dibutylsebacat, Triacetin, Rizinusöl, Glycerin oder Mischungen davon.

13. Die Zweischichtentablettenformulierung gemäß Anspruch 12, worin der Weichmacher Triethylcitrat ist.

14. Die pharmazeutische Formulierung gemäß einem der Ansprüche 1 bis 11, umfassend,
a. Flurbiprofen bei 5,0 - 25,0 % auf Tablettengesamtgewicht,
i. Amonium-Methacrylat-Copolymer zu 0,5 - 5,0 % auf Tablettengesamtgewicht,
ii. Polyethylenglycol zu 0,01 - 2,0 % auf Tablettengesamtgewicht,
iii. mikrokristalline Cellulose zu 1,0 - 15,0 % auf Tablettengesamtgewicht,
iv. Lactosemonohydrat zu 1,0 - 15,0 % auf Tablettengesamtgewicht,
v. Magnesiumstearat zu 0,01 - 2,0 % auf Tablettengesamtgewicht,
b. Glucosamin oder Salze davon zu 25,0 - 80,0 % auf Tablettengesamtgewicht,
i. Crosscarmellose-Natrium zu 0,5 - 5,0 % auf Tablettengesamtgewicht,
ii. Polyvinylpyrrolidon zu 0,5 - 5,0 % auf Tablettengesamtgewicht,
iii. mikrokristalline Cellulose zu 1,0 - 15,0 % auf Tablettengesamtgewicht,
iv. Lactosemonohydrat zu 1,0 - 15,0 % auf Tablettengesamtgewicht,
v. kolloidales Siliziumdioxid zu 0,01 - 2,0 % auf Tablettengesamtgewicht,
vi. Magnesiumstearat zu 0,01 - 2,0 % auf Tablettengesamtgewicht.

15. Die pharmazeutische Formulierung gemäß den Ansprüchen 1 bis 13, umfassend,
a. Flurbiprofen bei 5,0 - 25,0 % auf Tablettengesamtgewicht,
i. Amonium-Methacrylat-Copolymer zu 0,5 - 5,0 % auf Tablettengesamtgewicht,
ii. Neutralpellet zu 1,0 - 10,0 % auf Tablettengesamtgewicht,
iii. Triethylcitrat zu 0,01 - 2,0 % auf Tablettengesamtgewicht,
iv. kolloidales Siliziumdioxid zu 0,01 - 2,0 % auf Tablettengesamtgewicht,
v. Polyethylenglycol und mikrokristalline Cellulose zu 1,0 - 15,0 % auf Tablettengesamtgewicht,
vi. Magnesiumstearat zu 0,01 - 2,0 % auf Tablettengesamtgewicht,
b. Glucosamin oder Salze davon zu 25,0 - 80,0 % auf Tablettengesamtgewicht,
i. Croscarmellose-Natrium zu 0,5 - 5,0 % auf Tablettengesamtgewicht,
ii. Polyvinylpyrrolidon zu 0,5 - 5,0 % auf Tablettengesamtgewicht,
iii. mikrokristalline Cellulose zu 1,0 - 15,0 % auf Tablettengesamtgewicht,
iv. Lactose-Monohydrat zu 1,0 - 15,0 % auf Tablettengesamtgewicht,
v. kolloidales Siliziumdioxid zu 0,01 - 2,0 % auf Tablettengesamtgewicht,
vi. Magnesiumstearat zu 0,01 - 2,0 % auf Tablettengesamtgewicht.

## Revendications

1. Une formulation de comprimé bicouche, comprenant :
a. une première couche comprenant du flurbiprofène, laquelle première couche permettant une libération retardée du flurbiprofène,
b. une seconde couche comprenant de la glucosamine ou des sels de cette dernière, laquelle seconde couche permettant une libération immédiate de la glucosamine, dans laquelle ledit comprimé bicouche est en forme de sandwich.

2. La formulation de comprimé bicouche selon la revendication 1, dans laquelle ladite première couche comprend en outre des polymères de contrôle de vitesse sélectionnés dans le groupe comprenant les polyméthacrylates, en particulier les copolymères méthacrylate d'ammonium, le copolymère acide méthacrylate-acrylate d'éthyle, le copolymère acide méthacrylique-méthacrylate de méthyle, le méthacrylate cationique, le polyéthylène glycol, le phtalate acétate de cellulose, le monoglycéride acétylé, le tartrate de dibutyle, le phtalate de diéthyle, le phtalate de diméthyle, la glycérine, le propylène glycol et la triproprionine ou leurs mélanges.

3. La formulation de comprimé bicouche selon la revendication 2, dans laquelle lesdits polymères de contrôle de vitesse sont présents en une quantité allant de 0,5 à 5,0 % par rapport au poids total du comprimé.

4. La formulation de comprimé bicouche selon les revendications 2 et 3, dans laquelle lesdits polymères de contrôle de vitesse sont des copolymères de méthacrylate d'ammonium.

5. La formulation de comprimé bicouche selon la revendication 1, dans laquelle ladite seconde couche comprend en outre des désintégrants choisis dans le groupe comprenant la croscarmellose de sodium, la crospovidone, l'hydroxypropyl-cellulose faiblement substituée (L-HPC), le glycolate d'amidon de sodium, le xylitol, la (1-éthenpyrrolidin-2-one)-polyplasdone ou des mélanges de ces composés.

6. La formulation de comprimé bicouche selon la revendication 5, dans laquelle lesdits désintégrants sont présents en une quantité allant de 0,5 à 5,0 % par rapport au poids total du comprimé.

7. La formulation de comprimé bicouche selon les revendications 5 et 6, dans laquelle ledit désintégrant est le croscarmellose de sodium.

8. La formulation de comprimé bicouche selon la revendication 1, dans laquelle le flurbiprofène est présent en une quantité allant de 5,0 à 25,0 % par rapport au poids total de comprimé et la glucosamine ou ses sels en une quantité allant de 25,0 à 80,0 % par rapport au poids total du comprimé.

9. La formulation de comprimé bicouche selon l'une quelconque des revendications précédentes, dans laquelle lesdites couches comprennent en outre des excipients pharmaceutiquement acceptables choisis dans le groupe comprenant les liants, les charges, les agents de glisse, les lubrifiants ou des mélanges de ces composés.

10. La formulation pharmaceutique selon la revendication 9, dans laquelle ledit liant est choisi dans le groupe comprenant la polyvinylpyrrolidone (povidone), l'hydroxy-propyl-méthyl-cellulose, l'hydroxypropyl-cellulose, la carboxy-méthyl-cellulose, la méthyl-cellulose, l'hydroxy-éthyl-cellulose, la carboxy-méthyl-cellulose de sodium, la carboxy-méthyl-cellulose de calcium, l'éthyl-cellulose, le polyéthylène oxyde, la gélatine, l'amidon, la gomme xanthane, la gomme de guar, une alginate, le carragéenan, la pectine, les carbomères, l'acétate phtalate de cellulose, l'hydroxy-propyl-amidon, les polaxomères, le polyéthylène glycol ou des mélanges de ces composés.

11. La formulation de comprimé bicouche selon la revendication 10, dans laquelle ledit liant est la polyvinylpyrrolidone (povidone).

12. La formulation pharmaceutique selon l'une quelconque des revendications précédentes, dans laquelle la première couche peut comprendre un plastifiant qui est choisi dans le groupe comprenant les citrates d'ester tels que le citrate d'acétyle et de tributyle, le citrate d'acétyle triéthyle ou le citrate de triéthyle ; des esters de phtalate tels que le phtalate de diéthyle ou le phtalate de dibutyle ; des esters d'acide gras tels que le stéarate de butyle, le monostéarate de glycérol ou l'alcool stéarylique ; le sébaçate de dibutyle, la triacétine, l'huile de ricin, la glycérine ou des mélanges de ces composés.

13. La formulation de comprimé bicouche selon la revendication 12, dans laquelle ledit plastifiant est le citrate de triéthyle.

14. La formulation pharmaceutique selon les revendications 1 à 11, comprenant :
a. du flurbiprofène à raison de 5,0 à 25,0 % par rapport au poids total du comprimé,
i. un copolymère de méthacrylate d'ammonium à raison de 0,5 à 5,0 % par rapport au poids total du comprimé,
ii. du polyéthylène glycol à raison de 0,01 à 2,0 % par rapport au poids total du comprimé,
iii. de la cellulose microcristalline à raison de 1,0 à 15,0 % par rapport au poids total du comprimé,
iv. du monohydrate de lactose à raison de 1,0 à 15,0 % par rapport au poids total du comprimé,
v. du stéarate de magnésium à raison de 0,01 à 2,0 % par rapport au poids total du comprimé,
b. de la glucosamine ou des sels de glucosamine à raison de 25,0 à 80,0 % par rapport au poids total de comprimé,
i. de la croscarmellose de sodium à raison de 0,5 à 5,0 % par rapport au poids total du comprimé,
ii. de la polyvinylpyrrolidone à raison de 0,5 à 5,0 % par rapport au poids total du comprimé,
iii. de la cellulose microcristalline à raison de 1,0 à 15,0 % par rapport au poids total de comprimé,
iv. du monohydrate de lactose à raison de 1,0 à 15,0 % par rapport au poids total du comprimé,
v. du dioxyde de silicium colloïdal à raison de 0,01 à 2,0 % par rapport au poids total du comprimé,
vi. du stéarate de magnésium à raison de 0,01 à 2,0 % par rapport au poids total du comprimé.

15. La formulation pharmaceutique selon les revendications 1 à 13, comprenant :
a. du flurbiprofène à raison de 5,0 à 25,0 % par rapport au poids total du comprimé,
i. du copolymère méthacrylate d'ammonium à raison de 0,5 à 5,0 % par rapport au poids total du comprimé,
ii. des pellets neutres à raison de 1,0 à 10,0 % par rapport au poids total du comprimé,
iii. du citrate de triéthyle à raison de 0,01 à 2,0 % par rapport au poids total du comprimé,
iv. du dioxyde de silicium colloïdal à raison de 0,01 à 2,0 % par rapport au poids total du comprimé,
v. du polyéthylène glycol et de la cellulose microcristalline à raison de 1,0 à 15,0 % par rapport au poids total du comprimé,
vi. du stéarate de magnésium à raison de 0,01 à 2,0 % par rapport au poids total du comprimé,
b. de la glucosamine ou des sels de celle-ci à raison de 25,0 à 80,0 % par rapport au poids total du comprimé,
i. de la croscarmellose de sodium à raison de 0,5 à 5,0 % par rapport au poids total du comprimé,
ii. de la polyvinylpyrrolidone à raison de 0,5 à 5,0 % par rapport au poids total du comprimé,
iii. de la cellulose microcristalline à raison de 1,0 à 15,0 % par rapport au poids total du comprimé,
iv. du monohydrate de lactose à raison de 1,0 à 15,0 % par rapport au poids total du comprimé,
v. du dioxyde de silicium colloïdal à raison de 0,01 à 2,0 % par rapport au poids total du comprimé,
vi. du stéarate de magnésium à raison de 0,01 à 2,0 % par rapport au poids total du comprimé.
